# EUROPEAN PATENT SPECIFICATION

(11) **EP 1 297 741 B1**
(45) Date of publication and mention of the grant of the patent: **26.04.2006**
(21) Application number: 02078769.3
(22) Date of filing: 16.09.2002
(51) Int. Cl.: A01J 5/007, A01J 5/013

(54) **A device for milking animals**
Vorrichtung zum Melken von Tieren
Dispositif de traite des animaux

(30) Priority: 28.09.2001 NL 1019062
(43) Date of publication of application: 02.04.2003
(73) Proprietor: Lely Enterprises AG, 6300 ZUG (CH)
(72) Inventor: Vijverberg, Helena G. M., 3141 GH Maassluis (NL); Espada Aventin, Elena, 2627 AD Delft (NL)
(74) Representative: Corten, Maurice Jean F.M.

(56) References cited:
- EP-A- 1 000 535
- WO-A-01/19170
- WO-A-97/47187
- WO-A-99/31965
- US-A- 5 416 417

## Description

The present invention relates to a device for milking a dairy animal according to the preamble of claim 1.

Such a device is known from EP-A-1000535. The device known therefrom is provided with a measuring device in the form of a colour measuring system provided with one or more sensors comprising one or more sources irradiating the milk successively or simultaneously with radiation of one or more different wavelengths and/or different intensities, while, during at least a part of the time when the sources are in their switched-on position, one or more receivers establish the radiation intensity during a time interval. When the obtained measurement data indicate that the colour of the measured milk deviates from normal values, the relevant milk is separated. The colour measuring system may also be used for determining the quantity of air in the measured quantity of milk. However, it has appeared that the known device sometimes draws a wrong conclusion on the basis of the colour measurements, so that e.g. suitable milk is not used for being processed further, but is discharged.

It is i.a. an object of the invention to provide a device for milking a dairy animal by means of which the decision whether or not milk obtained is suitable for being processed further can be taken in an accurate manner.

According to the invention, for that purpose a device of the above-described type comprises the measures according to the characterizing part of claim 1. It has appeared that the presence of air, constituting a first milk variable, in the portion of the milk of which the value of the further milk variable (consequently not being air) is measured affects the reliability of the measurement, and consequently the correctness of the decision whether or not the milk obtained should be processed further. By establishing the presence of air in the mentioned portion and comparing it with a threshold, it is thus possible to judge measured values obtained at the presence of air in that portion of the milk in a different manner than measured values obtained at the absence of air in the milk line. Thus it is possible to take a more correct decision whether or not milk obtained should be processed further. Moreover, on the basis of the measurement signals it is possible to obtain a more accurate indication about the animal's health. The processing device preferably comprises the comparing device. It is noticed that as a threshold a lower threshold and/or an upper threshold may be taken. Further the value of the threshold may be predetermined, or be updated regularly or continuously. Further by 'issued to the processing device' is also meant 'processed by the processing device'.

In particular the processing device processes the measurement signal in dependence on the value of the air presence signal. Thus it may be ensured that measurement signals obtained at the presence of air in the milk line, i.e. unreliable measurement signals, are not processed or that there is first provided a compensation for the air present before the measurement signals are processed, so that only reliable measurement signals are taken into account for determining whether or not milk obtained should be processed further.

In an embodiment of a device according to the invention the aerometer comprises an air flow sensor. Alternatively or additionally the aerometer comprises a conductivity meter for measuring the conductivity of the mentioned portion of the milk. Alternatively or additionally the aerometer comprises a vacuum meter. Such meters are known per se and give a reliable indication of the presence of air and the extent to which air is present in the mentioned portion of the milk.

In a further embodiment of a device according to the invention the measuring device is suitable for measuring, during the entire course of the milking run, the value of the further milk variable for obtaining a measurement pattern of the further milk variable, and a memory of the processing device is preferably suitable for storing the measurement pattern. By not only using a certain value, but the entire pattern i.e. the course of the variable during the milking run for determining whether or not milk obtained should be processed further, it is possible to take a still more accurate decision whether or not the milk obtained should be processed further. Comparing measurement patterns with reference patterns appears to result in more correct decisions than exclusively comparing one single measured value.

The processing device is in particular provided with an averaging device for determining the average of a measurement pattern of a further milk variable, it being advantageous when the memory is suitable for storing the average measurement pattern. Such an average measurement pattern may excellently be used for determining deviations from this average pattern, which may be an indication that the condition of the dairy animal is different from normal or that the milk produced by the dairy animal is different from normal. Such an average measurement pattern appears to provide per animal a more accurate indication of the deviation than a predetermined reference value. Especially when the average is a so-called progressive average, i.e. an average over e.g. the last ten milking runs (another number is possible as well), the possibility of taking a correct decision is provided.

In an embodiment of a device according to the invention the memory is suitable for storing a reference pattern.

Although for all animals the same thresholds may be used, it is advantageous when the memory of the processing device contains an upper threshold pattern and/or a lower threshold pattern for a relevant measurement pattern of a further milk variable for an animal.

In a further embodiment of a device according to the invention the processing device is provided with a comparing device for comparing a momentary measurement pattern of a further milk variable with the stored measurement pattern of the further milk variable, and for issuing a comparison signal indicative of the comparison result. It is thus possible, when the device is provided with a milk line system comprising a number of lines and with at least one device controlled by the comparison signal for guiding milk flowing through the milk line system to a relevant line, to discharge automatically unsuitable milk or to convey suitable milk for being processed further.

For the purpose of enabling visual checking it is advantageous when the device comprises a displaying device for displaying the comparison signal. When the device comprises a device for generating a warning, said warning device being controlled by the comparison signal, it is possible, in certain situations, to give a warning to the manager of the device, e.g. in the form of a sound signal.

The measuring device preferably comprises a colour sensor measuring system for measuring the intensity of at least one wavelength band, in particular in the visible wavelength range of the milk obtained from the dairy animal, the variable being the intensity of the wavelength band. Especially with the aid of the colour sensor measuring system the intensity of the separate colours in the milk obtained from the separate udder quarters is established. Accordingly, in this embodiment the variable is constituted by the colour of the milk obtained.

In an embodiment of a device according to the invention the measuring device is constituted by a flow sensor for measuring the flow of the milk obtained during the milking run. The flow sensor preferably measures the flow of the milk obtained from the separate udder quarters.

In a further embodiment of a device according to the invention the measuring device is constituted by a conductivity meter known per se for measuring the conductivity of the milk obtained during the milking run. The conductivity meter preferably measures the conductivity of the milk obtained from the separate udder quarters.

In a still further embodiment of a device according to the invention the measuring device is constituted by a thermometer for measuring the temperature of the milk obtained during the milking run. The thermometer preferably measures the temperature of the milk obtained from the separate udder quarters.

In another further embodiment of a device according to the invention the measuring device is constituted by a component meter for measuring the quantity of a component of the milk obtained during the milking run, such as fat, protein, urea, bacteria, sugars, free fatty acids, germs, etc. The component meter preferably measures the components of the milk obtained from the separate udder quarters.

In a further embodiment of a device according to the invention the device is provided with a means for determining the period between two successive milking runs of the dairy animal, and the memory is suitable for containing a reference pattern in dependence on the measured period, respectively an upper threshold pattern and/or a lower threshold pattern in dependence on the measured period. This embodiment of the invention is based on the insight that the measured value of the variable depends on the period elapsed since the last milking run of the dairy animal, also called interval, even when the condition of the dairy animal remains unchanged. By including, according to the invention, various reference values for the variable in the memory, the reference values depending on the measured period, a more accurate comparison of the measured values is possible, so that it is possible to take a correct decision whether or not the milk is suitable for being processed further. Moreover, after comparison of the measured values with the reference values it is possible to draw more correct conclusions in relation to the condition respectively the health of the dairy animal. Measurement of the period may take place by using a clock measuring the period of time between two successive milking runs. Alternatively the number of cows having been milked since the last milking run of the relevant dairy animal may be an indication of the period. Consequently the reference values depend for example on the measured period of time or on the number of cows having been milked since the last milking run of the relevant animal, or on other variables comprising a time aspect.

The invention will be explained hereinafter in further detail with reference to an embodiment shown in the drawing, in which:
Figure 1 is a schematic view of a device for milking a cow, provided with a colour sensor measuring system, and
Figure 2 is a schematic view of a milking box with a milking robot provided with means for measuring a variable in relation to the cow.

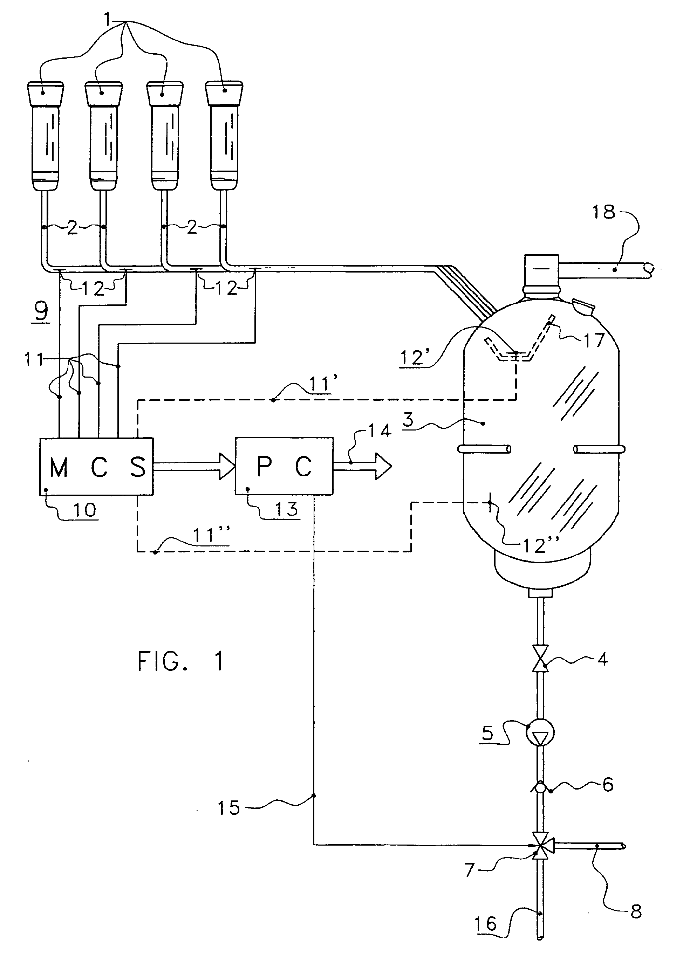

Figure 1 shows four teat cups 1 to be connected to the teats of an animal to be milked, the milk discharge lines 2 of said teat cups 1 debouching into a milk glass 3. To the milk glass 3 there is further connected a vacuum line 18 for the purpose of applying a vacuum in the milk glass 3 itself, in the milk discharge lines 2 and in the teat cups 1, said vacuum being required for keeping the teat cups connected to the teats of the animal, for milking and for separating milk and air present therein from each other in the milk glass 3. From the milk glass 3 the milk obtained is discharged via a valve 4, a pump 5, a non-return valve 6 and a three-way valve 7 through a line 8 to a not further shown milk tank.

Figure 1 further shows a colour sensor measuring system 9, said measuring system comprising a colour intensity processing unit (MCS) 10, to which four sensors 12 are connected via glass fibre cables 11. Said sensors 12 are disposed in the milk lines 2 for establishing the intensity of a number of defined colours in the milk and for supplying signals representing these intensities to the processing unit 10. As a colour sensor measuring system may be used the Modular Color Sensor system CS1 of Stracon Messsysteme GmbH, Im Camisch 10, Kahla. The sensors used in this system are sensitive to frequencies in frequency bands for red (R), green (G) and blue (B). Therefore there are issued three signals per measurement, which may be considered as intensity values for these three colours.

Although until now the opinion prevailed that for milk of a constant composition these three intensity values have a fixed mutual relation, said relation depending i.a. on the impurities and components in the milk, it has appeared that for certain dairy animals the relation between the three intensity values depends on the interval, in other words depends on the period between two successive milking runs. This period may be a period of time or a period depending on other variables, such as in particular the number of cows having been milked since the last milking run of the relevant cow.

The colour intensity processing unit (MCS) 10 comprises a computer (PC) 13 (shown in the figure separately from the colour intensity processing unit (MCS) for the sake of clearness), in which for each animal to be milked there is a file in which all data required for milking a relevant animal are stored.

During the entire course of a milking run also the obtained three intensity values of the relevant colours in the milk are stored. These intensity values stored at each milking run thus form a colour measurement pattern. The progressive average may be determined from the colour measurement patterns obtained for a certain animal during a defined number (e.g. ten, but an other number is also possible) of the last milking runs carried out. Upon averaging preferably milking runs with equal intervals are used. The colour patterns obtained at a next milking run with an equal interval may be compared with this progressive average colour measurement pattern, i.e. the last obtained colour measurement pattern of each of the three colours may be compared with the corresponding colour measurement pattern (preferably belonging to an equal interval), recorded in the computer as a progressive average. In other words, the colour measurement patterns are compared both mutually and with corresponding colour measurement patterns, recorded during one or more previous milking runs (preferably with an equal interval). This comparison process takes place in the computer 13 which also functions as a comparing device. Subsequently the results of this comparison process may be displayed on a displaying device in such a manner that the presence of certain substances, such as impurities, in the milk can be read directly therefrom. These results may be supplied via the line 14 to a screen or a printer.

Instead of determining the progressive average of the colour measurement pattern for each of the colours, it is also possible to determine in another manner for each colour a calibration pattern, such as in particular a reference pattern, respectively a lower threshold pattern or an upper threshold pattern. It is possible to apply calibration patterns which could hold for the milk obtained from all the animals or from a group of animals. In that case it will not be necessary to dispose a sensor 12 in each of the milk discharge lines 2, but an overflow reservoir 17 may be disposed in the milk glass 3, in which overflow reservoir there is provided such a sensor 12' which is connected to the processing unit 10 via a glass fibre cable shown by a "dashed" line 11'. As a further alternative a sensor 12" may be disposed in the lower part of the milk glass 3. Also in the latter case said sensor has to be connected to the processing unit 10 via a glass fibre cable 11".

However, in all situations it holds that, when inadmissible quantities of undesired substances appear to be present in the milk, the computer 13 issues a signal over the line 15 to the three-way valve 7, via which three-way valve 7 and the discharge line 16 connected thereto the milk containing these undesired substances may be discharged separately.

When for example blood has come into the milk, the colour measurement pattern issued by the sensor 12 for the colour red, will be a different pattern than when no blood is present in the milk. This colour measurement pattern will then be higher than the colour measurement pattern based on the progressive average or higher than the calibration pattern applied (preferably in dependence on the comparison with patterns belonging to the same interval). Also when there are no impurities in the milk, alterations in the concentration of substances normally being present in the milk may still be established during the milking run. It has further been found that the colour measurement patterns for the three colours have a mutually different ratio for different animals. Therefore it is advantageous to determine the colour measurement patterns for each animal separately at each milking run and to compare them with calibration patterns or, in particular, with progressive average colour measurement patterns established for this specific animal (and preferably belonging to the same interval).

An example of the dependence of the measured colour intensity (and consequently of the measured colour pattern) on the interval, said dependence having been proved clearly by means of the above-mentioned colour sensor measuring system, is given hereinafter. It has further appeared that this dependence is reproducible. For a particular cow it has appeared that the intensity of the blue frequency band rises in a particular manner when the period of time, the interval, increases. Likewise the intensity rises when more cows have been milked since the last milking run. It has further appeared that the intensity of the green frequency band shows a certain, slight fall at an increasing interval. The intensity of the red frequency band showed a certain slight rise. For this cow the total sum of the intensities appeared to rise to a maximum value at an increasing interval and to fall via a particular pattern at a further increasing interval. The value of the intensity in the red frequency band reduced by the value of the blue frequency band appeared to show with this cow a falling pattern at an increasing interval, whereas the quotient of the intensity in the red frequency band and the intensity in the green frequency band rose to a maximum value at an increasing interval and remained constant at a further increase of the interval. It will be obvious that upon comparing the milk obtained from this cow, at each interval there has to be taken a different reference value or pattern to decide whether or not the milk obtained is suitable for being processed further.

It has further appeared that the colour intensity may differ per quarter, so that it is advantageous to compare the colour measurement patterns per animal, per quarter and preferably per interval, in order to be able to decide whether or not milk obtained from a quarter should be processed further.

It has further appeared that the flow pattern of the milk obtained during the milking run is different per animal, and is further interval-dependent. Also here, to be able to take a correct decision whether or not the milk obtained should be processed further, the measured flow pattern has to be compared with a reference pattern for that interval. It is noticed that a flow sensor for measuring the flow of the milk obtained during the milking run is known per se. In particular the flow sensor measures the flow pattern of the milk obtained from the separate udder quarters.

It has further appeared that the conductivity pattern over the entire milking run may be different per animal or per group of animals, and may provide a more accurate decision whether or not the milk obtained should be processed further than only one single measured value. Besides, the conductivity of the milk obtained for the mentioned cow rises at an increasing interval. A conductivity meter for measuring the conductivity pattern of the milk obtained during the milking run, in particular per quarter, may then be used to take a correct decision whether or not the milk obtained (possibly per quarter) should be processed further.

It has further appeared that the temperature of the milk obtained for the mentioned cow rises at an increasing interval. In that situation a thermometer may be used for measuring the temperature pattern of the milk obtained during the milking run, in particular for measuring the temperature pattern of the milk obtained from the separate udder quarters, in order to take a correct decision whether or not the milk obtained (possibly per quarter and/or per interval) should be processed further.

Moreover it has appeared that for the mentioned cow the fat content of the milk obtained falls according to a certain curve at an increasing interval. Also for other components there appears to be a dependence between the quantity and the interval. A component meter for measuring the quantity pattern of a component of the milk obtained during the milking run, such as fat, protein, urea, bacteria, sugars, free fatty acids, germs, etc., in particular the component pattern of the milk obtained from the separate udder quarters, may then be used for taking a correct decision whether or not the milk obtained (possibly per quarter and/or per interval) should be processed further.

The above-mentioned relations have not only been found with a particular cow, but all cows appear to produce milk of which the measurable variables show a cow-dependent pattern. A particular pattern for one cow may then indicate milk suitable for being processed further, whereas the same pattern measured on milk obtained from another cow may indicate milk which is not suitable for being processed further.

A normal measurement pattern may be a predetermined reference pattern, or an average measurement pattern (preferably per interval) for an animal. For that purpose there is provided an averaging device for determining the average of a measurement pattern of a further milk variable. Besides, other reference patterns are possible as well (e.g. an upper threshold pattern and/or a lower threshold pattern).

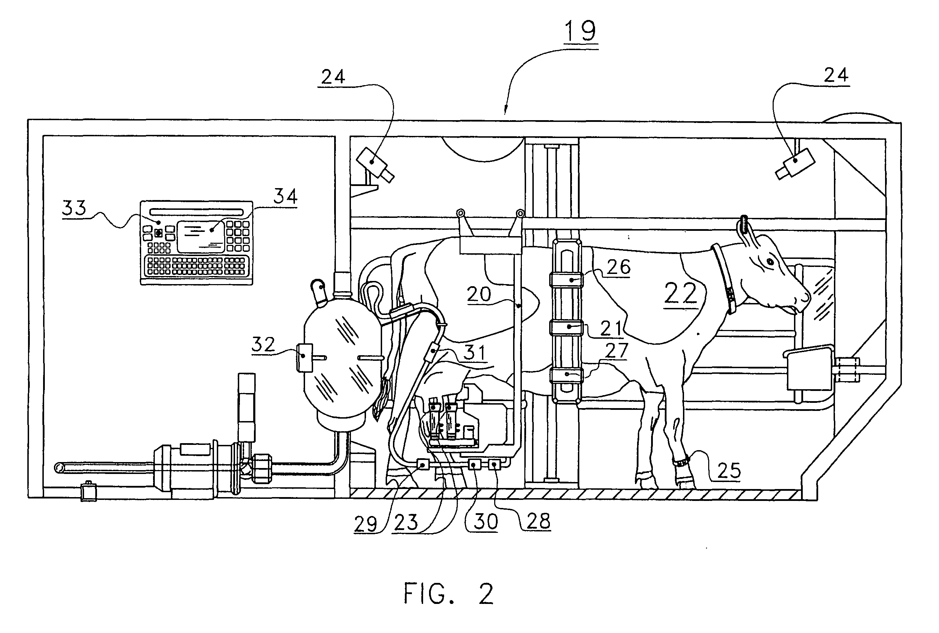

Figure 2 shows schematically a milking box 19 with a milking robot 20, to which the invention is in particular applicable. Said figure shows schematically various measuring devices for measuring the pattern of the values of variables in relation to the cow.

For the purpose of measuring the health of the cow 22, further the heart beat is measured by means of a band 21 including a heart beat meter around the leg or the abdomen of the cow 22. Alternatively or additionally a heart beat meter known per se may be provided on the cow 22 near a place where an artery is located, in this connection the udder or an ear of the cow may be taken into consideration. A suitable heart monitoring system is for example obtainable at Polar Electro Oy, Helsinki, Finland. Alternatively a heart beat meter may be included in at least one of the teat cups 23.

In the milking box 19 there may be disposed one or more cameras 24 for observing and measuring the activity of the cow 22, which cameras may also be used for monitoring the health of the cow 22. The video pictures are analysed by movement recognition equipment known per se for determining activity parameters such as stepping, kicking and the like. To that end the picture is compared per cow 22 with stored historical data regarding the cow 22. There may further be provided a step counter 25, a muscle contraction meter 26 and/or a muscle vibration meter 27 for determining the activity of the cow 22. Besides, the milk yield is measured by a quantity meter 32 i.e. a yield meter.

A flow sensor 28 measures the flow pattern of the milk obtained during a milking run. A conductivity meter 29 measures the conductivity pattern of the milk obtained during a milking run. A thermometer 30 measures the temperature pattern of the milk obtained during a milking run. A component meter 31 measures the component quantity pattern, e.g. protein and fat, in the milk obtained during the milking run. All these measurement data are transmitted to or read by a processing device 33 comprising a computer having a memory. Besides the measurement data the processing device 33 preferably also stores the period of time elapsed since the same animal has been milked, respectively stores the number of cows having been milked since the last milking run. To that end the processing device 33 comprises a clock (not explicitly shown, but implicitly present in the computer) for determining the period of time between two successive milking runs of the dairy animal. Alternatively the processing device may comprise a counter for counting the number of cows since the last milking run of the relevant cow. In the memory of the computer of the processing device 33 reference patterns are stored per interval, per animal or per group of animals, possibly per quarter, and per milk variable, respectively generated by the system itself. The processing device 33 comprises a (non-shown) comparing device for comparing the measured pattern of the variable with the stored reference patterns. The comparing device issues a comparison signal, the value of which depends on the comparison result, and is consequently indicative of the comparison result. This comparison signal may be displayed on a displaying device, such as a screen 34. As described above, the comparison signal may also be used for controlling a valve or the like, so that the milk obtained will be processed further or not. Should the comparison signal indicate a deviation, then it is also possible for the comparison signal to control a device for generating a warning (such as e.g. a loudspeaker) for issuing a signal (e.g. a sound) which is perceptible by a manager of the device.

It will be obvious that the measurement patterns may be used separately, but that also combinations of measurement patterns of different variables may be used for determining whether or not milk should be processed further (or for determining whether the condition of a dairy animal is within the standards). Thus a weight factor may be given to certain parameters or comparison results for combining the measurement patterns obtained in a desired manner.

As described, Figure 2 shows a side view of a milking box 19 with a cow 22 present therein. The milking box 19 is provided with a milking robot 20 with teat cups 23 which are automatically connected to the teats of the cow 22 by means of the milking robot 20. Near the front side of the milking box 19 there is further disposed a feeding trough to which concentrate may be supplied in metered quantities. Other elements of the milking box and the robot are not shown in the figure for the sake of clearness.

For the purpose of exclusively using correct measurement signals when deciding whether or not further to process milk obtained, according to the invention the presence of air is established in the portion of the milk of which also the value of the relevant variable is determined. Such air, in dependence on the extent thereof, appears to be able to disturb the measurements in an undesired manner. The presence of air in the mentioned portion of the milk may be established in a manner known per se by an air flow sensor and/or a conductivity meter and/or a vacuum meter. Such a meter provides a so-called air presence signal which may possibly indicate the quantity of air. Because such meters are known per se, a further description thereof is omitted. When such a meter detects air or detects that a certain to be pre-adjusted minimum quantity of air has been exceeded in the mentioned portion of the milk, the air presence signal may control the processing device in such a manner that the measured values of the further milk variable are not processed, for example are not stored in the memory, or are first compensated prior to being processed further. Thus unreliable measurements are either not taken into account when deciding whether or not milk obtained should be processed and/or should not be taken into account when determining the average measurement pattern for the further milk variable or unreliable measurements are first compensated for the presence of air.

When air is present in the mentioned portion of the milk, a warning may be given to the manager of the device. This manager may still decide after checking whether the measured values should be considered as correct values or should be deleted. To that end, at the presence of air, such measured values may be stored, e.g. in a separate memory. By pre-adjusting a minimum threshold of air it may be ensured that when the air present in the mentioned portion of the milk exceeds said minimum value, these measured values are deleted automatically. Automatic deletion of measured values measured at the presence of air in the milk line is possible, although as a result thereof correct measured values may be deleted in some cases in an undesired manner.

## Claims

1. A device for milking a dairy animal, such as a cow, said device being provided with a milk line (2), with a processing device (33), with an aerometer for determining the presence of air, constituting a first milk variable, in a portion of the milk flowing through the milk line (2) during a milking run of a dairy animal and for issuing an air presence signal to the processing device (33), with a measuring device for measuring a value of at least one further milk variable of the mentioned portion of the milk, the measuring device being suitable for generating a measurement signal indicative of the measured value of the further milk variable and for issuing the measurement signal to the processing device, **characterized in that** the device is provided with a comparing device for comparing the air presence signal with a threshold and for issuing a comparison signal, the measurement signal from the measuring device being issued to the processing device (33) in dependence on the comparison signal.

2. A device as claimed in claim 1, **characterized in that** the processing device comprises the comparing device.

3. A device as claimed in claim 1 or 2, **characterized in that** the aerometer comprises an air flow sensor.

4. A device as claimed in any one of the preceding claims, **characterized in that** the aerometer comprises a conductivity meter (29) for measuring the conductivity of the mentioned portion of the milk.

5. A device as claimed in any one of the preceding claims, **characterized in that** the aerometer comprises a vacuum meter.

6. A device as claimed in any one of the preceding claims, **characterized in that** the measuring device is suitable for measuring, during the entire course of the milking run, the value of the further milk variable for obtaining a measurement pattern of the further milk variable.

7. A device as claimed in claim 6, **characterized in that** the processing device (33) is provided with an averaging device for determining the average of a measurement pattern of a further milk variable.

8. A device as claimed in claim 7, **characterized in that** a memory of the processing device is suitable for storing the average measurement pattern.

9. A device as claimed in claim 6, 7 or 8, **characterized in that** a memory of the processing device is suitable for storing a reference pattern.

10. A device as claimed in any one of the preceding claims 6 through 9, **characterized in that** a memory of the processing device (33) contains an upper threshold pattern and/or a lower threshold pattern for a relevant measurement pattern of a further milk variable for a dairy animal.

11. A device as claimed in any one of the preceding claims 6 through 10, **characterized in that** the processing device is provided with a comparing device for comparing a momentary measurement pattern of a further milk variable with the stored measurement pattern of the further milk variable, and for issuing a comparison signal indicative of the comparison result.

12. A device as claimed in claim 11, **characterized in that** the device is provided with a milk line system comprising a number of milk lines (2) and with at least one device controlled by the comparison signal for guiding milk flowing through the milk line system to a relevant line.

13. A device as claimed in claim 11 or 12, **characterized in that** the device comprises a displaying device for displaying the comparison signal.

14. A device as claimed in claim 11, 12 or 13, **characterized in that** the device comprises a device for generating a warning, said warning device being controlled by the comparison signal.

15. A device as claimed in any one of the preceding claims, **characterized in that** the measuring device comprises a colour sensor measuring system (9) for measuring the intensity of at least one wavelength band, in particular in the visible wavelength range of the milk obtained from the dairy animal, the variable being the intensity of the wavelength band.

16. A device as claimed in claim 15, **characterized in that** with the aid of the colour sensor measuring system (9) the intensity of the separate colours in the milk obtained from the separate udder quarters is established.

17. A device as claimed in any one of the preceding claims, **characterized in that** the measuring device is constituted by a flow sensor (28) for measuring the flow of the milk obtained during the milking run.

18. A device as claimed in claim 17, **characterized in that** the flow sensor (28) measures the flow of the milk obtained from the separate udder quarters.

19. A device as claimed in any one of the preceding claims, **characterized in that** the measuring device is constituted by a conductivity meter (29) for measuring the conductivity of the milk obtained during the milking run.

20. A device as claimed in claim 19, **characterized in that** the conductivity meter (29) measures the conductivity of the milk obtained from the separate udder quarters.

21. A device as claimed in any one of the preceding claims, **characterized in that** the measuring device is constituted by a thermometer (30) for measuring the temperature of the milk obtained during the milking run.

22. A device as claimed in claim 21, **characterized in that** the thermometer (30) measures the temperature of the milk obtained from the separate udder quarters.

23. A device as claimed in any one of the preceding claims, **characterized in that** the measuring device is constituted by a component meter (31) for measuring the quantity of a component of the milk obtained during the milking run, such as fat, protein, urea, bacteria, sugars, free fatty acids, germs, etc.

24. A device as claimed in claim 23, **characterized in that** the component meter (31) measures the components of the milk obtained from the separate udder quarters.

25. A device as claimed in any one of the preceding claims, **characterized in that** the device is provided with a means for determining the period between two successive milking runs of the dairy animal, and **in that** the memory is suitable for containing a reference pattern in dependence on the measured period, respectively an upper threshold pattern and/or a lower threshold pattern in dependence on the measured period.

26. A device as claimed in claim 25, **characterized in that** the means for determining the period comprises a clock for measuring the period of time between two successive milking runs.

27. A device as claimed in claim 25, **characterized in that** the means is constituted by a counter for counting the number of dairy animals having been milked since the last time the relevant dairy animal was milked.

## Patentansprüche

1. Vorrichtung zum Melken eines milchgebenden Tieres, wie z. B. einer Kuh, wobei die Vorrichtung mit einer Milchleitung (2), mit einem Verarbeitungsgerät (33), mit einem Aerometer, um die Präsenz von Luft, die eine erste Melkvariable bildet, in einem Teil der Milch zu ermitteln, die während eines Melkdurchganges eines milchgebenden Tieres durch die Milchleitung (2) strömt, und um ein Luftpräsenzsignal an das Verarbeitungsgerät (33) zu geben, sowie mit einer Meßvorrichtung zum Messen eines Wertes mindestens einer weiteren Melkvariablen des genannten Teiles der Milch, wobei die Meßvorrichtung dazu ausgelegt ist, ein Meßsignal zu erzeugen, das Aufschluß über den gemessenen Wert der weiteren Melkvariablen gibt, und das Meßsignal an das Verarbeitungsgerät zu liefern,
**dadurch gekennzeichnet, daß** die Vorrichtung mit einer Vergleichsvorrichtung versehen ist, um das Luftpräsenzsignal mit einem Grenzwert zu vergleichen und ein Vergleichssignal auszugeben, wobei das Meßsignal von der Meßvorrichtung in Abhängigkeit von dem Vergleichssignal an das Verarbeitungsgerät (33) geliefert wird.

2. Vorrichtung nach Anspruch 1,
**dadurch gekennzeichnet, daß** das Verarbeitungsgerät die Vergleichsvorrichtung umfaßt.

3. Vorrichtung nach Anspruch 1 oder 2,
**dadurch gekennzeichnet, daß** das Aerometer einen Luftströmungssensor umfaßt.

4. Vorrichtung nach einem der vorhergehenden Ansprüche,
**dadurch gekennzeichnet, daß** das Aerometer einen Leitfähigkeitsmesser (29) zum Messen der Leitfähigkeit des genannten Teiles der Milch umfaßt.

5. Vorrichtung nach einem der vorhergehenden Ansprüche,
**dadurch gekennzeichnet, daß** das Aerometer einen Vakuummesser umfaßt.

6. Vorrichtung nach einem der vorhergehenden Ansprüche,
**dadurch gekennzeichnet, daß** die Meßvorrichtung dazu ausgelegt ist, während der gesamten Dauer des Melkdurchganges den Wert der weiteren Melkvariablen zu messen, um ein Meßmuster der weiteren Melkvariablen zu erzielen.

7. Vorrichtung nach Anspruch 6,
**dadurch gekennzeichnet, daß** das Verarbeitungsgerät (33) mit einer Vorrichtung zur Ermittlung des Mittelwertes versehen ist, um den Mittelwert eines Meßmusters einer weiteren Melkvariablen zu ermitteln.

8. Vorrichtung nach Anspruch 7,
**dadurch gekennzeichnet, daß** ein Speicher des Verarbeitungsgerätes dazu ausgelegt ist, das Durchschnittsmeßmuster zu speichern.

9. Vorrichtung nach Anspruch 6, 7 oder 8,
**dadurch gekennzeichnet, daß** ein Speicher des Verarbeitungsgerätes dazu ausgelegt ist, ein Bezugsmuster zu speichern.

10. Vorrichtung nach einem der vorhergehenden Ansprüche 6 bis 9,
**dadurch gekennzeichnet, daß** ein Speicher des Verarbeitungsgerätes (33) ein Muster für den oberen Grenzwert und/oder ein Muster für den unteren Grenzwert für ein jeweiliges Meßmuster einer weiteren Melkvariablen für ein milchgebendes Tier enthält.

11. Vorrichtung nach einem der vorhergehenden Ansprüche 6 bis 10,
**dadurch gekennzeichnet, daß** das Verarbeitungsgerät mit einer Vergleichsvorrichtung zum Vergleichen eines momentanen Meßmusters einer weiteren Melkvariablen mit dem gespeicherten Meßmuster der weiteren Melkvariablen und zum Ausgeben eines Vergleichssignals versehen ist, das Aufschluß über das Vergleichsergebnis gibt.

12. Vorrichtung nach Anspruch 11,
**dadurch gekennzeichnet, daß** die Vorrichtung mit einem Milchleitungssystem versehen ist, das eine Anzahl von Milchleitungen (2) umfaßt, sowie mit mindestens einer von dem Vergleichssignal gesteuerten Vorrichtung, um durch das Milchleitungssystem fließende Milch zu einer entsprechenden Leitung zu leiten.

13. Vorrichtung nach Anspruch 11 oder 12,
**dadurch gekennzeichnet, daß** die Vorrichtung eine Anzeigevorrichtung zum Anzeigen des Vergleichssignals umfaßt.

14. Vorrichtung nach Anspruch 11, 12 oder 13,
**dadurch gekennzeichnet, daß** die Vorrichtung eine Vorrichtung zum Erzeugen eines Warnsignals umfaßt, wobei die Warnvorrichtung von dem Vergleichssignal gesteuert wird.

15. Vorrichtung nach einem der vorhergehenden Ansprüche,
**dadurch gekennzeichnet, daß** die Meßvorrichtung ein Farbsensormeßsystem (9) zum Messen der Intensität mindestens eines Wellenlängenbandes umfaßt, insbesondere im sichtbaren Wellenlängenbereich der von dem milchgebenden Tier gewonnenen Milch, wobei die Variable durch die Intensität des Wellenlängenbandes gebildet ist.

16. Vorrichtung nach Anspruch 15,
**dadurch gekennzeichnet, daß** mit Hilfe des Farbsensormeßsystems (9) die Intensität der einzelnen Farben in der von den einzelnen Eutervierteln gewonnenen Milch ermittelt wird.

17. Vorrichtung nach einem der vorhergehenden Ansprüche,
**dadurch gekennzeichnet, daß** die Meßvorrichtung durch einen Strömungssensor (28) zum Messen des während des Melkdurchganges gewonnenen Milchstromes gebildet ist.

18. Vorrichtung nach Anspruch 17,
**dadurch gekennzeichnet, daß** der Strömungssensor (28) den Strom der von den einzelnen Eutervierteln gewonnenen Milch mißt.

19. Vorrichtung nach einem der vorhergehenden Ansprüche,
**dadurch gekennzeichnet, daß** die Meßvorrichtung durch einen Leitfähigkeitsmesser (29) zum Messen der Leitfähigkeit der während des Melkdurchganges gewonnenen Milch gebildet ist.

20. Vorrichtung nach Anspruch 19,
**dadurch gekennzeichnet, daß** der Leitfähigkeitsmesser (29) die Leitfähigkeit der von den einzelnen Eutervierteln gewonnenen Milch mißt.

21. Vorrichtung nach einem der vorhergehenden Ansprüche,
**dadurch gekennzeichnet, daß** die Meßvorrichtung durch ein Thermometer (30) zum Messen der Temperatur der während des Melkdurchganges gewonnenen Milch gebildet ist.

22. Vorrichtung nach Anspruch 21,
**dadurch gekennzeichnet, daß** das Thermometer (30) die Temperatur der von den einzelnen Eutervierteln gewonnenen Milch mißt.

23. Vorrichtung nach einem der vorhergehenden Ansprüche,
**dadurch gekennzeichnet, daß** die Meßvorrichtung durch ein Bestandteilsmeßgerät (31) gebildet ist, um die Menge eines Bestandteiles, wie z. B. Fett, Protein, Harnstoff, Bakterien, Zucker, freie Fettsäuren, Keimgehalt, etc., der während des Melkdurchganges gewonnenen Milch zu messen.

24. Vorrichtung nach Anspruch 23,
**dadurch gekennzeichnet, daß** das Bestandteilsmeßgerät (31) die Bestandteile der von den einzelnen Eutervierteln gewonnenen Milch mißt.

25. Vorrichtung nach einem der vorhergehenden Ansprüche,
**dadurch gekennzeichnet, daß** die Vorrichtung mit einer Vorrichtung versehen ist, um den Abschnitt zwischen zwei aufeinanderfolgenden Melkdurchgängen des milchgebenden Tieres zu ermitteln, und daß der Speicher dazu ausgelegt ist, in Abhängigkeit von dem gemessenen Abschnitt ein Bezugsmuster bzw. in Abhängigkeit von dem gemessenen Abschnitt ein Muster für den oberen Grenzwert und/oder ein Muster für den unteren Grenzwert zu speichern.

26. Vorrichtung nach Anspruch 25,
**dadurch gekennzeichnet, daß** die Vorrichtung zum Ermitteln des Abschnittes eine Uhr zum Messen des Zeitabschnittes zwischen zwei aufeinanderfolgenden Melkdurchgängen umfaßt.

27. Vorrichtung nach Anspruch 25,
**dadurch gekennzeichnet, daß** die Vorrichtung durch einen Zähler zum Zählen der Anzahl von milchgebenden Tieren gebildet ist, die seit dem letzten Melken des betreffenden milchgebenden Tieres gemolken worden sind.

## Revendications

1. Dispositif de traite d'un animal laitier, tel qu'une vache, ledit dispositif étant pourvu d'un lactoduc (2), d'un dispositif de traitement (33), d'un aéromètre destiné à déterminer la présence d'air, constituant une première variable de lait, dans une partie de l'écoulement du lait à travers le lactoduc (2) pendant une traite d'un animal laitier et à délivrer un signal de présence d'air au dispositif de traitement (33), d'un dispositif de mesure destiné à mesurer une valeur d'au moins une autre variable de lait de la partie mentionnée du lait, le dispositif de mesure convenant pour générer un signal de mesure indiquant la valeur mesurée de l'autre variable de lait et délivrer le signal de mesure au dispositif de traitement, **caractérisé en ce que** le dispositif est pourvu d'un dispositif de comparaison destiné à comparer le signal de présence d'air à un seuil et à délivrer un signal de comparaison, le signal de mesure provenant du dispositif de mesure étant délivré au dispositif de traitement (33) en fonction du signal de comparaison.

2. Dispositif selon la revendication 1, **caractérisé en ce que** le dispositif de traitement comprend le dispositif de comparaison.

3. Dispositif selon la revendication 1 ou 2, **caractérisé en ce que** l'aéromètre comprend un capteur de flux.

4. Dispositif selon l'une quelconque des revendications précédentes, **caractérisé en ce que** l'aéromètre comprend un conductivimètre (29) destiné à mesurer la conductivité de la partie mentionnée du lait.

5. Dispositif selon l'une quelconque des revendications précédentes, **caractérisé en ce que** l'aéromètre comprend un vacuomètre.

6. Dispositif selon l'une quelconque des revendications précédentes, **caractérisé en ce que** le dispositif de mesure convient pour mesurer, pendant l'intégralité de la traite, la valeur de l'autre variable de lait pour obtenir un modèle de mesure de l'autre variable de lait.

7. Dispositif selon la revendication 6, **caractérisé en ce que** le dispositif de traitement (33) est pourvu d'un dispositif de formation de moyenne destiné à former la moyenne d'un modèle de mesure d'une autre variable de lait.

8. Dispositif selon la revendication 7, **caractérisé en ce qu'**une mémoire du dispositif de traitement convient pour mémoriser le modèle de mesure de moyenne.

9. Dispositif selon la revendication 6, 7 ou 8, **caractérisé en ce qu'**une mémoire du dispositif de traitement convient pour mémoriser un modèle de référence.

10. Dispositif selon l'une quelconque des revendications 6 à 9, **caractérisé en ce qu'**une mémoire du dispositif de traitement (33) contient un modèle seuil supérieur et/ou un modèle seuil inférieur pour un modèle de mesure correspondant d'une autre variable de lait d'un animal laitier.

11. Dispositif selon l'une quelconque des revendications 6 à 10, **caractérisé en ce que** le dispositif de traitement est pourvu d'un dispositif de comparaison destiné à comparer un modèle de mesure momentané d'une autre variable de lait au modèle de mesure mémorisé de l'autre variable de lait, et à délivrer un signal de comparaison indiquant le résultat de la comparaison.

12. Dispositif selon la revendication 11,
**caractérisé en ce que** le dispositif est pourvu d'un système de lactoduc comprenant un certain nombre de lactoducs (2) et d'au moins un dispositif commandé par le signal de comparaison destiné à guider le lait s'écoulant à travers le système de lactoduc en direction d'une conduite correspondante.

13. Dispositif selon la revendication 11 ou 12, **caractérisé en ce que** le dispositif comprend un dispositif d'affichage destiné à afficher le signal de comparaison.

14. Dispositif selon la revendication 11, 12 ou 13, **caractérisé en ce que** le dispositif comprend un dispositif destiné à générer une alerte, ledit dispositif d'alerte étant commandé par le signal de comparaison.

15. Dispositif selon l'une quelconque des revendications précédentes, **caractérisé en ce que** le dispositif de mesure comprend un système de mesure à capteur de couleur (9) destiné à mesurer l'intensité d'au moins une bande de longueur d'onde, en particulier dans la plage de longueur d'onde visible du lait obtenu de l'animal laitier, la variable étant l'intensité de la bande de longueur d'onde.

16. Dispositif selon la revendication 15, **caractérisé en ce que** l'intensité des couleurs séparées dans le lait obtenu à partir des quartiers de pis séparés est établie à l'aide du système de mesure à capteur de couleur (9).

17. Dispositif selon l'une quelconque des revendications précédentes, **caractérisé en ce que** le dispositif de mesure est constitué par un capteur de flux (28) destiné à mesurer le flux du lait obtenu pendant la traite.

18. Dispositif selon la revendication 17, **caractérisé en ce que** le capteur de flux (28) mesure le flux du lait obtenu à partir des quartiers de pis séparés.

19. Dispositif selon l'une quelconque des revendications précédentes, **caractérisé en ce que** le dispositif de mesure est constitué par un conductivimètre (29) destiné à mesurer la conductivité du lait obtenu pendant la traite.

20. Dispositif selon la revendication 19, **caractérisé en ce que** le conductivimètre (29) mesure la conductivité du lait obtenu à partir des quartiers de pis séparés.

21. Dispositif selon l'une quelconque des revendications précédentes, **caractérisé en ce que** le dispositif de mesure est constitué par un thermomètre (30) destiné à mesurer la température du lait obtenu pendant la traite.

22. Dispositif selon la revendication 21, **caractérisé en ce que** le thermomètre (30) mesure la température du lait obtenu à partir des quartiers de pis séparés.

23. Dispositif selon l'une quelconque des revendications précédentes, **caractérisé en ce que** le dispositif de mesure est constitué par un dispositif de mesure de composant (31) destiné à mesurer la quantité d'un composant du lait obtenu pendant la traite, tel que la graisse, les protéines, l'urée, les bactéries, les sucres, les acides gras libres, les germes, etc.

24. Dispositif selon la revendication 23, **caractérisé en ce que** le dispositif de mesure de composant (31) mesure les composants du lait obtenu à partir des quartiers de pis séparés.

25. Dispositif selon l'une quelconque des revendications précédentes, **caractérisé en ce que** le dispositif est pourvu de moyens destinés à déterminer la période entre deux traites successives de l'animal laitier, et **en ce que** la mémoire convient pour contenir un modèle de référence en fonction de la période mesurée, respectivement un modèle seuil supérieur et/ou un modèle seuil inférieur en fonction de la période mesurée.

26. Dispositif selon la revendication 25, **caractérisé en ce que** les moyens destinés à déterminer la période comprennent une horloge destinée à mesurer la période de temps entre deux traites successives.

27. Dispositif selon la revendication 25, **caractérisé en ce que** les moyens sont constitués par un compteur destiné à compter le nombre d'animaux laitiers ayant été traits depuis la dernière fois où l'animal laitier correspondant a été trait.
